# DEMANDE DE BREVET EUROPEEN

(11) **EP 0 630 966 A1**
(43) Date de publication de la demande: **28.12.1994**
(21) Numéro de dépôt: 94401383.8
(22) Date de dépôt: 20.06.1994
(51) Int. Cl.: C12M 1/04, C02F 3/28

(54) **Installation perfectionnée de fermentation anaérobie de matières organiques**

(30) Priorité: 23.06.1993 FR 9307648
(71) Demandeur: GAZ DE FRANCE, F-75017 Paris (FR)
(72) Inventeur: Fichet, Georges, F-92160 Antony (FR)
(74) Mandataire: Durand, Yves Armand Louis

(57) **Abrégé**

La présente invention concerne une installation perfectionnée de fermentation anaérobie de matières organiques.

Cette installation comprend une pluralité de buses (3, 4, 5) d'injection sous pression de biogaz dans un fermenteur (1), lesquelles buses sont implantées sur le pourtour du fermenteur pour améliorer l'écoulement des matières dans ce fermenteur ainsi que son rendement, et sont alimentées en biogaz par une canalisation en forme de deux demi-couronnes (8, 9) entourant le fermenteur (1) et raccordées à une alimentation (11) en biogaz.

Cette installation permet de résoudre les problèmes de transit et de fermentation des matières organiques dans des fermenteurs de volume important.

## Description

La présente invention a essentiellement pour objet une installation perfectionnée de fermentation anaérobie de matières organiques provenant par exemple de déchets urbains tels que des ordures ménagères.

On sait depuis longtemps que les installations de fermentation anaérobie de matières organiques comprennent essentiellement un fermenteur dans lequel on fait séjourner un certain temps les matières organiques dans le but de produire notamment du biogaz.

Toutefois le fermenteur, surtout lorsqu'il présente un volume important, pose des problèmes d'écoulement des matières qu'il contient.

En effet, dans les fermenteurs de gros volume, de nombreux phénomènes rhéologiques interviennent tant au niveau de l'introduction des matières que de leur transit dans les fermenteurs.

Pendant le temps de séjour de la matière dans le fermenteur, il est très difficile, même à l'aide d'injection verticale à travers la masse dans le fermenteur de biogaz recyclé et destiné à déplacer cette masse de l'entrée vers la sortie du fermenteur, de maintenir une viscosité homogène dans la totalité de cette masse.

Dans les zones périphériques à l'intérieur du fermenteur, il reste notamment des quantités de matière difficiles à contrôler.

Dans ces zones, du fait de l'immobilité de la matière, la viscosité évolue par rapport au reste du milieu, des cisaillements de couches se produisent, et il en résulte une ségrégation des différents composants. Les particules les plus lourdes cheminent vers le bas du fermenteur et créent un dépôt.

Une telle sédimentation, si rien n'est fait, est irréversible et évolutive. Les dépôts augmentent et réduisent de façon continue le volume actif du fermenteur et par conséquent son rendement.

Etant donné qu'un fermenteur est mis en service pour de nombreuses années, il est de première importance de maîtriser ce phénomène, c'est-à-dire, en bref, de résoudre le problème de l'écoulement des matières dans les fermenteurs surtout lorsque ces derniers présentent un gros volume.

La présente invention parvient à ce but.

A cet effet, elle a pour objet une installation perfectionnée de fermentation anaérobie de matières organiques, telles que par exemple des ordures ménagères, dans un fermenteur où séjournent un certain temps lesdites matières dans le but de produire notamment du biogaz, caractérisée par une pluralité de buses d'injection sous pression de biogaz et/ou autres matières gazeuses, liquides, ou plus ou moins solides, dans le fermenteur, lesquelles buses sont réparties sur le pourtour du fermenteur suivant un même plan ou des plans différents pour ainsi améliorer l'écoulement des matières dans le fermenteur et son rendement surtout lorsque celui-ci présente un volume important.

Suivant une autre caractéristique de cette installation, les buses d'injection solidaires de la paroi du fermenteur sont raccordées par l'intermédiaire d'une vanne à au moins une canalisation en couronne entourant le fermenteur et elle-même raccordée à au moins une conduite d'alimentation en biogaz recyclé ou en une autre matière.

Suivant un mode de réalisation préféré, cette installation est caractérisée en ce que la canalisation précitée forme deux demi-couronnes réunies par seulement une de leurs extrémités pour ainsi constituer une zone de raccordement comportant deux vannes entre lesquelles est raccordée la conduite d'alimentation précitée.

Cette installation est encore caractérisée en ce que certaines buses d'injection sont implantées sensiblement normalement à la paroi du fermenteur, tandis que d'autres buses sont implantées sur la paroi du fermenteur suivant des directions non normales à ladite paroi.

Selon encore une autre caractéristique de l'invention, le fermenteur étant de forme cylindrique circulaire, comporte plusieurs groupes de buses d'injection situées dans au moins un plan de section transversale droite du fermenteur, et à savoir :
- un premier groupe de buses implantées sur la paroi externe du fermenteur symétriquement de part et d'autre d'un premier diamètre du fermenteur à une distance à partir de ce diamètre égale à un tiers du rayon du fermenteur, l'axe de ces buses étant orienté radialement ;
- un deuxième groupe de buses implantées sur la paroi externe du fermenteur symétriquement de part et d'autre d'un deuxième diamètre du fermenteur, perpendiculaire au premier diamètre, l'axe de ces buses formant un angle d'environ 15° avec la direction centrifuge du deuxième diamètre ; et
- un troisième groupe de buses implantées sur la paroi externe du fermenteur au point d'intersection de la paroi avec une ligne droite médiatrice du rayon du premier diamètre, l'axe de chaque base de ce troisième groupe coïncidant avec la corde de l'arc de paroi reliant ladite buse à la buse voisine du premier groupe de buses.

L'installation selon cette invention est encore caractérisée en ce que la conduite d'alimentation précitée des demi-couronnes raccordées aux buses est alimentée en biogaz par au moins une capacité reliée à un compresseur.

Mais d'autres caractéristiques et avantages de l'invention apparaîtront mieux dans la description détaillée qui suit et se réfère aux dessins annexés, donnés uniquement à titre d'exemple, et dans lesquels.

La figure 1 est une vue schématique et en coupe transversale d'un fermenteur équipé des moyens selon cette invention.

La figure 2 est une vue également schématique et en coupe transversale du fermenteur illustrant la position et l'orientation des buses d'injection, suivant un exemple de réalisation.

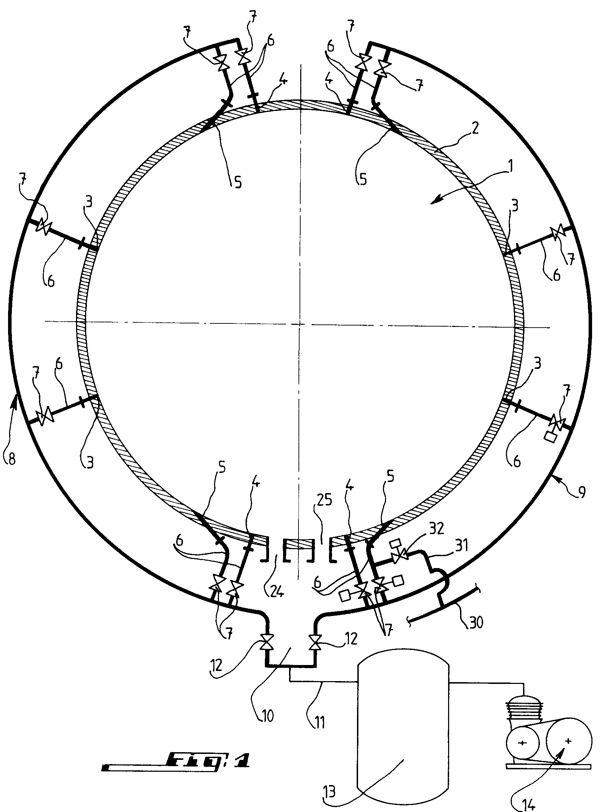

En se reportant plus particulièrement à la figure 1, on voit une installation de fermentation anaérobie se composant essentiellement d'un fermenteur ou digesteur 1 dans lequel peuvent être introduites des matières organiques qui y séjournent un certain temps et dont la fermentation permet de produire du biogaz.

Conformément à l'invention, une pluralité de buses 3, 4, 5 d'injection sous pression de biogaz et/ou autres matières gazeuses, liquides ou plus ou moins solides, sont implantées sur la paroi externe 2 du fermenteur 1.

Certaines de ces buses, telles que les buses 3 sont implantées sensiblement normalement à la paroi 2 du fermenteur 1, tandis que d'autres buses, telles que les buses 4 et 5 sont implantées sur la paroi 2 du fermenteur 1 suivant des directions non normales à la paroi 2.

Comme il apparaît clairement sur la figure 1, les buses d'injection 3, 4, 5 solidaires de la paroi 2 du fermenteur 1 sont raccordées par l'intermédiaire d'un conduit 6 avec vanne 7 à une canalisation en forme de deux demi-couronnes 8, 9 entourant le fermenteur 1.

Plus précisément, les deux demi-couronnes 8, 9 sont réunies par seulement une de leurs extrémités pour ainsi former une zone de raccordement 10 munie de deux vannes 12. Entre ces deux vannes, est raccordée une conduite 11 d'alimentation sous pression de biogaz recyclé. La conduite 11 est reliée à une capacité 13 elle même alimentée en biogaz par un compresseur 14. Ainsi, la capacité 6 pourra emmagasiner du biogaz jusqu'à l'obtention de la pression requise dans les demi-couronnes 8, 9 fournissant du gaz aux buses d'injection 3, 4, 5, via les conduits 6.

Dans certaines circonstances d'exploitation, il peut être avantageux, et cela sans sortir du cadre de l'invention, d'utiliser à la place du biogaz, de la matière organique fraîche ou prélevée sur un autre fermenteur, cette matière pouvant aussi bien sûr être injectée dans le fermenteur 1 via les buses 3, 4, 5. Une telle matière pourra être injectée par exemple via une couronne 30 indépendante de la canalisation 8, 9 et raccordée à un conduit 31 avec vanne 32 communiquant avec une ou des buses telles que 5.

Cette solution peut permettre de rétablir une dérive momentanée de l'homogénéité de l'ensemble de la matière dans le digesteur 1 et particulièrement dans les zones périphériques internes du digesteur. On pourra donc utiliser soit un système avec capacité 13 et compresseur 14 pour l'alimentation, ou bien un autre système, en fonction de la matière à injecter dans le digesteur pour résoudre les problèmes d'écoulement et de transit de ladite matière dans le digesteur, surtout lorsque celui-ci présente un gros volume.

Les buses d'injection 3, 4, 5 pourront avoir une orientation bien déterminée en fonction de la matière injectée par les buses et des conditions opératoires, comme on le décrira en détail ultérieurement.

Les vannes 7 en amont des buses d'injection 3, 4, 5 raccordées par les conduits 6 aux demi-couronnes 8, 9 pourront être pilotées à partir d'un automate ou autre appareil de programmation (non représenté), suivant une séquence appropriée et pouvant varier selon la viscosité de la matière fermentescible. Une telle séquence pourra être réalisée aussi bien lorsqu'on utilise du biogaz recyclé comme matière d'injection, que lorsqu'on utilise de la matière organique fraîche ou des matières à un niveau de dégradation intermédiaire prélevées sur un autre digesteur.

Les buses d'injection 3, 4, 5 seront évidemment judicieusement orientées de façon à permettre une homogénéisation de la totalité de la matière en fermentation dans le digesteur 1.

En outre, leur nombre pourra être quelconque, et elles pourront être réparties sur le pourtour du digesteur 1 suivant un même plan, ou suivant des plans différents sur toute la hauteur du digesteur. C'est dire, dans ce dernier cas, que des demi-couronnes 8, 9 assemblées comme expliqué précédemment pourront être installées à plusieurs niveaux le long de la hauteur du fermenteur 1, étant entendu que la prévision de demi-couronnes avec buses d'injection associées en partie basse du fermenteur 1 demeure la plus importante, puisque c'est là que les risques de sédimentation sont les plus marqués.

A la place d'une canalisation formée de deux demi-couronnes 8, 9 réunies comme montré sur la figure 1 et qui, à l'opposé de la zone de raccordement 10, présente une interruption, on pourrait parfaitement, sans sortir de la présente invention, utiliser une canalisation en forme de couronne unique entourant totalement la paroi externe 2 du fermenteur 1.

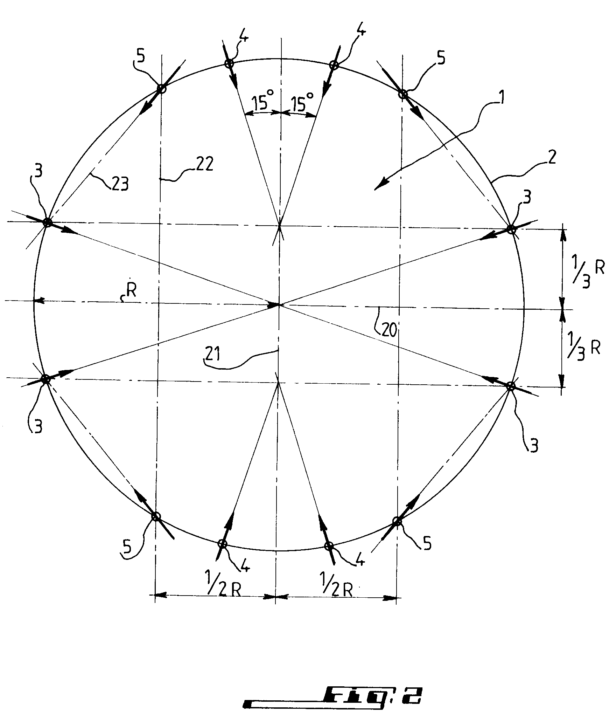

On se reportera maintenant à la figure 2 qui illustre clairement un exemple de réalisation d'implantation des buses d'injection 3, 4, 5 sur la paroi 2 du fermenteur 1, et cela dans un plan de section transversale droite dudit fermenteur.

Les buses d'injection 3, 4, 5 forment trois groupes de buses implantées et orientées de la façon suivante.

Le premier groupe se compose de quatre buses 3 qui sont implantées sur la paroi 2 du digesteur 1 symétriquement de part et d'autre d'un premier diamètre 20 du fermenteur et à une distance à partir de ce diamètre 20 égale à un tiers du rayon R du fermenteur. Comme on le voit bien sur la figure, l'axe des buses 3 est orienté radialement.

Le deuxième groupe se compose de quatre buses 4 qui sont implantées sur la paroi 2 symétriquement de part et d'autre d'un deuxième diamètre 21 du fermenteur, perpendiculaire au premier diamètre 20. L'axe de ces buses forme un angle de 15° avec la direction centrifuge du deuxième diamètre 20.

Enfin, le troisième groupe de buses se compose de quatre buses 5 implantées sur la paroi 2 du fermenteur au point d'intersection d'une ligne droite 22 médiatrice du rayon du premier diamètre 20. Comme on le voit bien sur la figure 2, l'orientation ou l'axe de chaque buse 5 coïncide avec la corde 23 de l'arc de paroi 2 reliant une buse 5 à une buse voisine 3 du premier groupe.

Selon cet exemple d'implantation, on utilise douze buses d'injection dont quatre, à savoir les buses 3 sont orientées radialement, tandis que les huit autres buses sont plus ou moins inclinées.

Mais on pourrait parfaitement utiliser un autre nombre de buses d'injection avec une orientation différente et cela, dans plusieurs plans répartis sur la hauteur du fermenteur, sans sortir du cadre de l'invention.

Le fermenteur 1 peut être un fermenteur de volume important et comporter, comme montré en 24 et 25 sur la figure 1, une entrée et une sortie de matière, ainsi qu'également une cloison (non représentée) coïncidant avec le deuxième diamètre 21, prenant racine sur la périphérie interne du fermenteur 1 entre l'entrée et la sortie 24, 25 et s'étendant sur seulement une partie du deuxième diamètre 21.

Bien entendu, l'invention n'est nullement limitée au mode de réalisation décrit et illustré qui n'a été donné qu'à titre d'exemple.

C'est ainsi que la position et l'orientation des buses d'injection peuvent être quelconques et sont fonction des dimensions du fermenteur et de la nature des matières à traiter. De même le nombre de demi-couronnes avec piquages d'injection peut être quelconque, tout comme le type de vannes utilisées en amont des injections, à la condition que l'ouverture, le temps d'ouverture et la fermeture de ces vannes puissent être commandés suivant une séquence quelconque par un automate programmable ou analogue. Les buses d'injection seront également d'une conception appropriée quelconque et aptes à permettre l'injection de gaz, liquides ou même matières plus ou moins solides.

C'est dire que l'invention comprend tous les équivalents techniques des moyens décrits ainsi que leurs combinaisons si celles-ci sont effectuées suivant son esprit.

## Revendications

1. Installation perfectionnée de fermentation anaérobie de matières organiques, telles que par exemple des ordures ménagères, dans un fermenteur (1) où séjournent un certain temps lesdites matières dans le but de produire notamment du biogaz, caractérisée par une pluralité de buses (3, 4, 5) d'injection sous pression de biogaz et/ou autres matières gazeuses, liquides ou plus ou moins solides, dans le fermenteur (1), lesquelles buses sont réparties sur le pourtour du fermenteur (1) suivant un même plan ou des plans différents pour ainsi améliorer l'écoulement des matières dans le fermenteur et son rendement surtout lorsque celui-ci présente un volume important.

2. Installation selon la revendication 1, caractérisée en que les buses d'injection (3, 4, 5) solidaires de la paroi (2) du fermenteur (1) sont raccordés par l'intermédiaire d'une vanne (7) à au moins une canalisation en couronne entourant le fermenteur et elle-même raccordée à au moins une conduite (11) d'alimentation en biogaz recyclé ou en une autre matière.

3. Installation selon la revendication 1 ou 2, caractérisée en ce que la canalisation précitée forme deux demi-couronnes (8, 9) réunies par seulement une de leurs extrémités pour ainsi constituer une zone de raccordement (10) comportant deux vannes (12) entre lesquelles est raccordée la conduite d'alimentation précitée (11).

4. Installation selon l'une des revendications 1 à 3, caractérisée en ce que certaines buses d'injection (3) sont implantées sensiblement normalement à la paroi (2) du fermenteur (1), tandis que d'autres buses (4, 5) sont implantées sur la paroi (2) du fermenteur (1) suivant des directions non normales à ladite paroi.

5. Installation selon l'une quelconque des revendications précédentes, caractérisée en ce que le fermenteur (1) étant de forme cylindrique circulaire, comporte plusieurs groupes de buses d'injection situés dans au moins un plan de section transversale droite du fermenteur, et à savoir :
- un premier groupe de buses (3) implantées sur la paroi externe (2) du fermenteur (1) symétriquement de part et d'autre d'un premier diamètre (20) du fermenteur à une distance à partir de ce diamètre égale à un tiers de rayon du fermenteur (1) , l'axe de ces buses (3) étant orienté radialement ;
- un deuxième groupe de buses (4) implantées sur la paroi externe (2) du fermenteur (1) symétriquement de part et d'autre d'un deuxième diamètre (21) du fermenteur, perpendiculaire au premier diamètre (20), l'axe de ces buses formant un angle d'environ 15° avec la direction centrifuge du deuxième diamètre (21) ; et
- un troisième groupe de buses (5) implantées sur la paroi externe (2) du fermenteur (1) au point d'intersection de la paroi (2) avec une ligne droite (22à médiatrice du rayon du premier diamètre (20), l'axe de chaque buse (5) de ce troisième groupe coïncidant avec la corde (23) de l'arc de paroi reliant ladite buse (5) à la buse voisine (3) du premier groupe.

6. Installation selon l'une des revendications 1 à 5, caractérisée en ce que la conduite d'alimentation précitée (11) est alimentée en biogaz par une capacité (13) reliée à un compresseur (14).
